# EUROPÄISCHE PATENTANMELDUNG

(11) **EP 0 827 725 A1**
(43) Veröffentlichungstag der Anmeldung: **11.03.1998**
(21) Anmeldenummer: 96114292.4
(22) Anmeldetag: 06.09.1996
(51) Int. Cl.: A61F 2/06

(54) **Stent zum Anordnen in einer Körperröhre**

(71) Anmelder: WILLY RÜSCH AG, D-71394 Kernen (DE)
(72) Erfinder: Freitag Lutz Dr., 58675 Hemer (DE)
(74) Vertreter: Ksoll, Peter, Dr.-Ing.

(57) **Zusammenfassung**

Die Erfindung betrifft einen Stent zum Anordnen in einer Körperröhre mit einem Stützgerüst (2) aus Nitinol, welches in einen Mantel (3) aus einem Shape-Memory-Polyurethan eingebettet ist. Das Stützgerüst (2) ist durch Schlitzen und anschließendes Aufweiten einer Metallröhre gebildet. Die Schneidlinien sind so ausgeführt, daß sich ein Stützgerüst (2) ergibt mit mehreren zueinander parallelen Ringen (4, 4.1-4.3) aus endlos zick-zack-förmig aneinanderschließenden Schrägstreben (5, 5.1-5.4) gleicher Länge. Diese sind über sich parallel zur Stentachse (A) erstreckende Axialstreben (6, 6.1-6.3) verbunden sind. Die Axialstreben (6, 6.1-6.3) reichen jeweils von der Spitze (7) zweier V-förmig aneinanderschließender Schrägstreben (5.1, 5.2) eines Rings (4.1) in das Tiefste (8) zweier V-förmig aneinanderschließender Schrägstreben (5.3, 5.4) eines benachbarten Rings (4.2). Der Stent (1) ist längenstabil und verhält sich dämpfend bei kurzen Belastungen. Zeitlich länger anhaltenden Belastungen paßt sich der Stent (1) konfigurativ an.

## Beschreibung

Die Erfindung betrifft einen Stent zum Anordnen in einer Körperröhre gemäß den Merkmalen im Oberbegriff des Anspruchs 1.

Bei einem Stent handelt es sich um einen hohlzylindrisch ausgebildeten Körper, der operativ perkutan oder endoskopisch implantiert wird, um tubuläre Körperröhren offenzuhalten, die sich als Folge einer Stenose verengen. Ein solcher Stent muß mit seiner Rückstellkraft einer äußeren Kompression widerstehen, die beispielsweise durch einen Tumor oder einen Lymphknoten hervorgerufen wird oder sich durch eine Striktur infolge einer Verätzung oder Vernarbung ergibt.

Durch die EP 0 506 918 B1 ist ein Stent der im Oberbegriff des Anspruchs 1 beschriebenen Art bekannt. Das Stützgerüst dieses Stents besteht aus ringförmig ausgebildeten Grundelementen, welche eine in sich geschlossene Zick-Zack-Form besitzen. Untereinander sind die Grundelemente über sich parallel zur Stentachse erstreckende Axialstreben miteinander verbunden. Das Stützgerüst wird zusätzlich von einem maschenartigen Gitter ummantelt.

Der bekannte Stent ist fertigungstechnisch aufwendig. Die Grundelemente werden aus einem Draht geformt und die Enden durch eine Schweißverbindung aneinandergefügt. Anschließend werden die Grundelemente durch die Axialstreben miteinander verbunden. Diese Vorgehensweise erfordert einen hohen Arbeits- und Zeitaufwand und ist folglich kostenintensiv.

Desweiteren erfordert das Gitter, welches das Stützgerüst umhüllt, zusätzlichen Material- und Fertigungsaufwand.

Ferner ist zu bemängeln, daß der bekannte Stent aufgrund seiner geometrischen Konfiguration nicht längenstabil ist. Bei Einwirken einer äußeren Belastung verlängert sich der Stent und bei Entlastung verkürzt er sich entsprechend.

Dies ist zunächst bei der Plazierung des Stents in einer Körperröhre hinderlich. Besonders nachteilig macht sich dieser Umstand jedoch beim Einsatz in einer Körperröhre bemerkbar, die einer pulsierenden äußeren Belastung unterliegt, wie beispielsweise ein Blutgefäß oder die Luftröhre. Durch die pulsierende Querschnittsveränderung der Körperröhre kommt es beim Stent zu einer konsekutiven Längenänderung. Folglich bewegt sich der Stent frequenzabhängig ständig hin und her. Hierdurch kann es zu Relativbewegungen zwischen dem Stent und der Wand der Körperröhre kommen. Als Folge treten Reizungen der Röhrenwand oder des umliegenden Gewebes auf. Selbst ein Verrutschen des Stents ist möglich. Aus diesem Grund sind bei der bekannten Bauart bewegungshindernde Widerhaken vorgesehen. Diese können das Problem aber nicht beheben, da sie ihrerseits Reizungen im umliegenden Gewebe hervorrufen können und die mechanische Einwirkung auf die umliegende Röhrenwand bei pulsierenden Bewegungen sogar verstärken.

Der Erfindung liegt ausgehend vom Stand der Technik die Aufgabe zugrunde, einen Stent zu schaffen, bei dem bei einer äußeren lateralen oder zirkulären Belastung nahezu keine Längenveränderung auftritt bzw. der längenstabil ist und der darüberhinaus einfacher und wirtschaftlicher zu fertigen ist.

Die Lösung dieser Aufgabe besteht nach der Erfindung in den im Kennzeichen des Anspruchs 1 aufgeführten Merkmalen.

Danach wird das Stützgerüst von mehreren zueinander parallelen Ringen aus sich endlos zick-zack-förmig aneinanderschließenden Schrägstreben gebildet, die über Axialstreben gekoppelt sind. Dabei erstrecken sich die Axialstreben jeweils von der Spitze zweier V-förmig aneinandergeschlossener Schrägstreben des einen Ringes zum tiefsten zweier V-förmig aneinandergeschlossener Schrägstreben des benachbarten Rings.

Die Erfindung macht sich dabei die Erkenntnis zu eigen, daß jeweils zwei Schrägstreben und eine Axialstrebe ein Getriebe bilden, bei dem die sich durch äußere Belastung ergebenden Verschiebungen durch die Schrägstreben kompensiert werden und die Axialstreben lageorientiert bleiben. Hierdurch wird eine Längenstabilität des Stents erreicht, und zwar sowohl bei einer lateralen Krafteinwirkung als auch bei einer zirkulären Krafteinwirkung. Gleichzeitig gewährleistet die Konfiguration des Stützgerüstes eine ausreichende Rückstellkraft gegenüber der äußeren Belastung.

Der erfindungsgemäße Stent begegnet schnellen pulsierenden Querschnittsänderungen gering elastisch mit einem geringen inneren Verschiebeverhalten. Bei längerfristigen Striktionen hingegen, beispielsweise durch Tumore, gibt der Stent nach und paßt sich der Belastungskontur an. Mithin besitzt der Stent ein adaptives Verhalten bei langfristigen Einwirkungen, jedoch ein dämpfendes Verhalten bei kurzfristigen Belastungsschwankungen.

Wesentlich ist weiterhin, daß das Stützgerüst einstückig stoffschlüssig, d.h. ohne Fügemaßnahmen durch Schlitzen und anschließendes Aufweiten eines dünnwandigen metallisches Tubus gebildet ist.

Der erfindungsgemäße Stent ist daher besonders wirtschaftlich zu fertigen. Der Tubus wird mittels eines Lasers mit dem gewünschten Schnittmuster versehen. In dieser Ausgangsstelle liegen die Streben des Stützgerüstes in einer näherungsweisen Totlage ohne Zwischenraum aneinander. Anschließend wird der Tubus in dem erforderlichen Maß aufgeweitet. Die Schrägstreben können hierbei einen Winkel von größer 0° und und kleiner 90°, gemessen zur Stentlängsachse, einnehmen. Vorzugsweise liegt der Winkel der Schrägstreben zwischen 0° und 45°.

Durch eine entsprechende Auslegung mit Längenabstimmung von Schräg- und Axialstreben kann auch ein Stent hergestellt werden, der im Durchmesser variiert. Es sind unterschiedliche, nahezu beliebige Durchmesserkonfigurationen möglich. Dementsprechend kann beispielsweise ein eiförmiger, ein eingeschnürter oder doppelkonischer Stent realisiert werden.

Die bekannten tubulären Strukturen Luftröhre, Speiseröhre, Schlagadern, Saugadern, Gallengänge usw. haben völlig unterschiedliche Eigenschaften. Hierauf kann der Stent spezifisch abgestimmt und beispielsweise hinsichtlich Durchmesser, Rückstellkraft und Adaptionsverhalten angepaßt werden. Desweiteren kann der Stent zum Überbrücken von Defekten wie Fisteln oder Dehiszenzen und Aneurysmen eingesetzt werden.

Nach den Merkmalen des Anspruchs 2 sind die Axialstreben hintereinander angeordnet. Sie bilden dann durchgehende Längsträger, die durch V-Elemente aus zwei Schrägstreben verbunden sind. Auf diese Weise entsteht ein sehr gering elastisches Stützgerüst mit hoher Rückstellkraft.

Eine besonders vorteilhafte Ausführungsform der Erfindung ist in den Merkmalen des Anspruchs 3 zu sehen, wonach die Axialstreben eines Rings und die Axialstreben der benachbarten Ringe in Umfangsrichtung versetzt angeordnet sind.

Ein Ring eines Stützgerüsts setzt sich dann aus hintereinander angeordneten V-Elementen aus zwei Schrägstreben und Y-Elementen aus zwei Schrägstreben und einer Axialstrebe zusammen. Benachbarte Ringe sind jeweils um ein Element versetzt. Dieser Aufbau bildet ein in sich längenstabiles Gesamtsystem. Längenveränderungen des Stents bei einer lateralen oder zirkulären Krafteinwirkung treten nicht auf. Gleichzeitig bringt der erfindungsgemäße Stent einer äußeren Belastung ausreichende Rückstellkräfte entgegen.

Die V-Elemente und Y-Elemente können jeweils abwechselnd hintereinander angeordnet sein, es ist aber auch möglich, zwei oder mehrere V-Elemente aneinander zu ketten und dann ein Y-Element vorzusehen.

Eine den allgemeinen Erfindungsgedanken weiterbildende Ausführungsform ist in Anspruch 4 charakterisiert. Danach sind die Schrägstreben dünner als die Axialstreben ausgebildet und damit biegeweicher. Diese Ausbildung unterstützt die Getriebebewegung der Schrägstreben bei einer Kompression. Gleichzeitig bleiben die Axialstreben in ihrer Lage stabil.

Den Merkmalen des Anspruchs 5 entsprechend sind die Knotenpunkte zwischen den Schrägstreben und den Axialstreben materialmäßig verstärkt. Die Verstärkung gewährleistet eine Spannungskonstanz im Übergangsbereich. Materialbrüchen infolge der Biegewechselbelastungen wird so entgegengewirkt.

Mit den Merkmalen des Anspruchs 6 kann die Lageorientierung eines Stents in der Körperröhre positiv unterstützt werden. Hierzu sind am Mantel über den Umfang verteilt Vorsprünge vorgesehen. Diese können gleichmäßig oder unregelmäßig angeordnet sein.

Obwohl es grundsätzlich denkbar ist, die Vorsprünge auf unterschiedlichste Weise zu erzeugen, beispielsweise in Form von Noppen, wird eine für die Praxis besonders interessante Lösung darin gesehen, die Vorsprünge dadurch zu erzeugen, daß zwei V-förmig aneinanderschließende Schrägstreben nach außen gekippt oder tordiert werden. Auf diese Weise erhält die Oberfläche des Stents eine nicht glatte schuppenartige oder unregelmäßig geriffelte Widerlagerstruktur. Diese Ausbildung ist schonend für die umliegenden Gefäßwände und Schleimhäute, da ein Abschnüren oder Abdrücken vermieden wird.

Gemäß den Merkmalen des Anspruchs 7 besteht das Stützgerüst aus einem Memory-Metall. Besonders geeignet ist hierfür eine Nickel-Titan-Legierung (Nitinol). Hierbei handelt es sich um eine Formgedächtnislegierung. Dieses Material hat bei einer tiefen Temperatur eine komprimierte Struktur. Es dehnt sich jedoch bei Überschreiten einer Grenztemperatur aus. Die jeweils gewünschten Grenzbedingungen können durch eine entsprechende Wahl der Legierungskomponenten eingestellt werden.

Eine weitere vorteilhafte Ausführungsform wird nach den Merkmalen des Anspruchs 8 darin gesehen, das Stützgerüst in einen Mantel aus einem thermoplastischen Polymer einzubetten.

Der Mantel verhindert zunächst, daß das Stützgerüst von Gewebezellen durchwachsen wird. Desweiteren unterstützt der Mantel das Rückstellverhalten des Stents.

Grundsätzlich weist Silikon als Mantelwerkstoff gute Eigenschaften auf. In der Kombination mit einem Stützgerüst aus Memory-Metall muß der Mantel die Expansion des Stützgerüstes zulassen.

Für die Praxis besonders vorteilhafte Eigenschaften ergeben sich bei einem Mantel aus einem viskoelastischen Polymer. Messungen haben ergeben, daß ein solcher Stent der Einwirkung einer äußeren Kraft kurzfristig einen höheren Widerstand entgegensetzt, die nachfolgend zeitabhängig absinkt. Der Stent reagiert damit auf die Belastung unelastisch und dämpfend.

Umgekehrt baut der Stent Kraft auf, wenn er entlastet wird. Bei einer Abnahme der Kompression sinkt die Rückstellkraft des Stents zunächst entsprechend ab, steigt dann aber wieder über die Zeit an. Der Stent paßt sich so der Körperröhre konfigurativ wieder vollständig an.

Bei einem solchen viskoelastischen Polymer kann es sich um ein Memory-Elastomer handeln (Shape Memory Polyurethan). Dieses Material ist temperaturabhängig und im kalten Zustand hart und klein. Folglich kann der Stent problemlos eingeführt werden. Erst bei Körpertemperatur bzw. geringfügig unterhalb der normalen Körpertemperatur kommt es zur Expansion des Stents.

Insbesondere die Kombination eines Memory-Elastomers als Mantel mit einem Stützgerüst aus Memory-Metall wird als vorteilhaft angesehen. Das Stützgerüst ist dann bei Raumtemperatur weich, der Mantel hingegen hart. Durch die Kombination ergibt sich ein Stent, der bei Raumtemperatur dünn und hart ist. Er kann folglich problemlos positioniert werden. Hierzu trägt auch bei, daß Nitinol im kalten Zustand verformbar, das heißt plastisch weich ist.

Bei Körpertemperatur wird dann der Mantel weich und flexibel, während sich das Stützgerüst aus Nitinol entfaltet. Expandiert liegt dann ein harter aber elastischer Stent vor.

Die Kombination verschiedener Polymerwerkstoffe im Mantel, wie sie Anspruch 9 vorsieht, ermöglicht eine zusätzliche Variation im Belastungsverhalten eines Stents. Denkbar ist beispielsweise die Kombination von Silikon, thermoplastischem Polyurethan und/oder Memory-Polyurethan, die jeweils unterschiedliche Zeitkonstanten aufweisen. Dies ermöglicht eine noch weitere spezifische Anpassung eines Stents hinsichtlich seiner medizinischen Verwendung.

Beim Einsatz in einer Schlagader beispielsweise wird die flache Pulsation zugelassen, größeren Schwankungen jedoch elastisch eine Kraft entgegengesetzt. Beim Einsatz in einer Speiseröhre z.B. bleibt der Schluckakt unbeeinträchtigt, wohingegen einer tumorösen Kompression mit einer ausreichenden Gegenkraft begegnet wird.

Die geometrische Ausbildung der Abschnitte aus unterschiedlichem Polymerwerkstoff kann variieren. Sie können sowohl als Längsabschnitte als auch als Radialabschnitte oder Wendelabschnitte ausgebildet sein.

Die Erfindung ist nachfolgend anhand von in den Zeichnungen dargestellten Ausführungsbeispielen näher erläutert. Es zeigen:
- Figur 1: eine erste Ausführungsform eines erfindungsgemäßen Stents;
- Figuren 2 bis 4: in Ausschnittsdarstellungen drei Fertigungsstadien eines Stützgerüsts;
- Figur 5: eine weitere Ausführungsform eines Stents;
- Figur 6: in der Seitenansicht einen vertikalen Längsschnitt durch den Mantel eines Stents;
- Figur 7: einen Stent mit einem Mantel aus unterschiedlichen Polymerwerkstoffen;
- Figur 8: einen vergrößerten Ausschnitt aus einem Stützgerüst und
- Figur 9: das Kraft-Weg-Diagramm eines Stents mit einem Mantel aus Polyurethan.

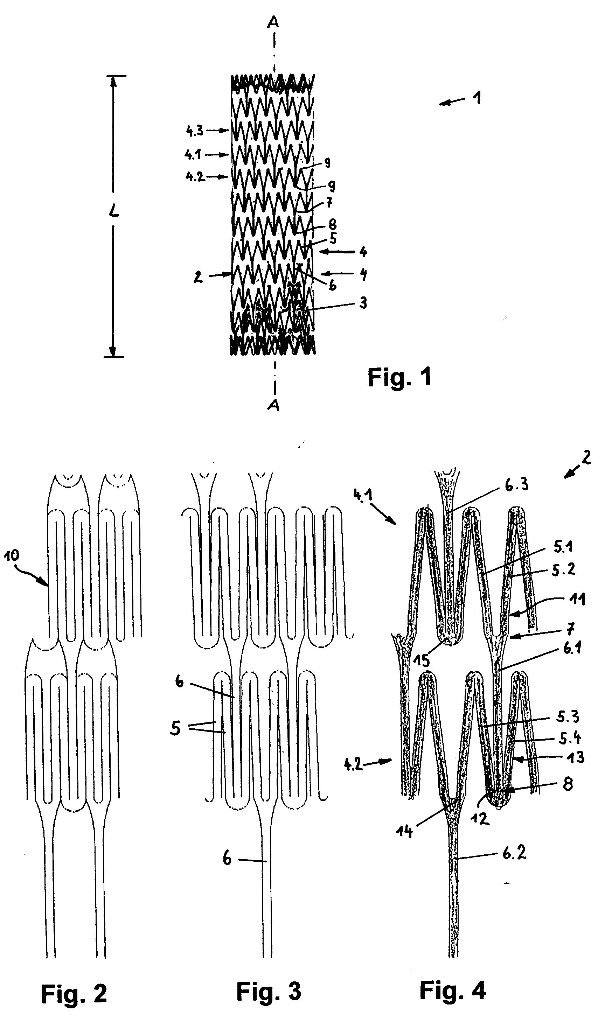

Die Figur 1 zeigt einen Stent 1 mit einem Stützgerüst 2 aus einer Nickel-Titan-Legierung und einem Mantel 3 aus Polyurethan.

Das Stützgerüst 2 ist durch Schlitzen eines Tubus mit Hilfe eines Lasers entsprechend eines vorgegebenen Schnittmusters und anschließendes Aufweiten über einen Dorn entstanden. Danach wird das Stützgerüst 2 mit flüssigem Polyurethan überzogen, welches nach dem Verfestigen den flexiblen Mantel 3 bildet. Der Mantel 3 ist in der Figur 1 grau schattiert angedeutet.

Das Stützgerüst 2 besteht aus mehreren zueinander parallelen Ringen 4. Die Ringe 4 werden von endlos zick-zack-förmig aneinanderschließenden Schrägstreben 5 gleicher Länge gebildet. Untereinander sind die Ringe 4 durch sich parallel zur Stentachse A liegende Axialstreben 6 verbunden.

Eine Axialstrebe 6.1 erstreckt sich jeweils von der Spitze 7 zweier V-förmig aneinanderschließender Schrägstreben 5.1, 5.2 eines Rings 4.1 in das Tiefste 8 zweier V-förmig aneinanderschließender Schrägstreben 5.3, 5.4 eines benachbarten Rings 4.2 (vgl. hierzu auch Figur 4).

Man erkennt weiterhin, daß die Axialstreben 6.1 des Rings 4.1 und die Axialstreben 6.2, 6.3 der jeweils benachbarten Ringe 4.2, 4.3 in Umfangsrichtung versetzt angeordnet sind.

Die Übergänge 9 zwischen den Schrägstreben 5 sind gerundet.

Bei einer äußeren Krafteinwirkung bleibt der Stent 1 über seine Länge L stabil. Verschiebungen werden durch die Schrägstreben 5, 5.1-5.4 kompensiert, indem diese eine Getriebebewegung ausführen und sich aufeinander zu bewegen, die Axialstreben 6, 6.1-6.3 aber in ihrer Lage orientiert bleiben. In seiner Gesamtheit ist der Stent 1 dann längenstabil.

Die Figuren 2 bis 4 zeigen an einem Ausschnitt die Vorgehensweise bei der Herstellung des Stützgerüsts 2.

Hierzu wird zunächst ein metallischer Tubus entsprechend einer vorgegebenen Schnittbahn 10, wie sie Figur 2 zeigt, geschlitzt. Dies ist vollautomatisch und mit hoher Genauigkeit mittels eines Lasers möglich. Auf diese Weise werden die Schrägstreben 5 und Axialstreben 6 erzeugt, die in Ausgangsstellung, wie sie Figur 3 zeigt, nahezu ohne Zwischenraum nebeneinander liegen.

Anschließend wird das Stützgerüst 2 aufgeweitet, so daß die Maschenkonfiguration wie in Figur 4 dargestellt entsteht. Das so hergestellte Stützgerüst 2 ist nahtlos ohne eine Fügemaßnahme hergestellt.

Wie die Figur 4 weiterhin verdeutlicht, bilden die V-förmig aneinanderschließenden Schrägstreben 5.1 und 5.2 zusammen mit der Axialstrebe 6.1 ein Y-Emelent 11. Dieses taucht mit seinem Fuß 12 in ein V-Element 13 des benachbarten Rings 4.2 ein. Das V-Element 13 wird durch die aneinandergeschlossenen Schrägstreben 5.3 und 5.4 gebildet.

In einem Ring 4.1 bzw. 4.2 reihen sich abwechselnd Y-Elemente 11 und V-Elemente 13 aneinander, wobei diese bei benachbarten Ringen 4.1, 4.2 in Umfangsrichtung jeweils um ein Element 11, 13 versetzt sind.

Die Knotenpunkte 14, 15 von Axialstreben 6 und Schrägstreben 5 sind materialmäßig verstärkt ausgebildet. Auf diese Weise wird einer Materialermüdung in diesen Übergangsbereichen entgegengewirkt, die bei einer äußerer Belastung Biegewechselbeanspruchungen unterliegen.

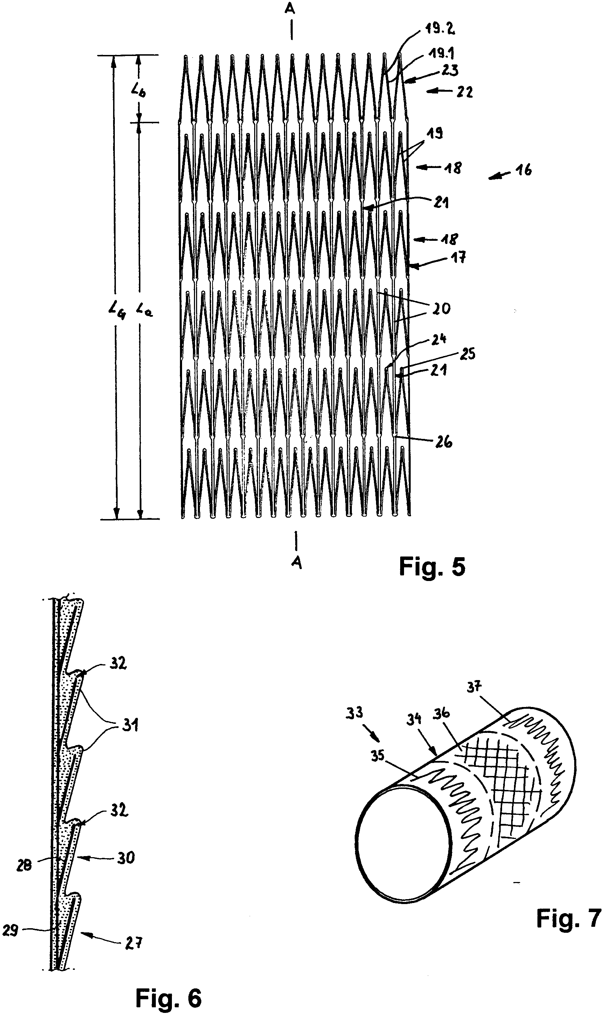

Eine andere Ausführungsform eines Stents 16 zeigt Figur 5. Das Stützgerüst 17 besteht wiederum aus Ringen 18 von sich V-förmig aneinanderschließenden Schrägstreben 19 und parallel zur Stentachse A angeordneten Axialstreben 20.

Die Axialstreben 20 sind hintereinander angeordnet, so daß sie auf einem Längenabschnitt Lₐ durchgehende Längsträger 21 bilden. An seinem Ende 22 schließt der Stent 16 mit einem Ring 23 aus Schrägstreben 19.1, 19.2 ab.

Zu einer Längenveränderung des Stents 16 bei einer äußeren Belastung kann es nur im Ring 23 auf dem Längenabschnitt L_{b} kommen. Eine hier auftretende Längenveränderung ist jedoch gegenüber der Gesamtlänge L_{G} von Stent 16 vernachlässigbar.

In der Figur 5 erkennt man ferner, daß die Schrägstreben 19 dünner als die Axialstreben 20 ausgebildet sind. Hierdurch sind die Schrägstreben 19 biegeweicher und reagieren bei einer äußeren Krafteinwirkung mit einer Kompensationsbewegung, ohne daß die Axialstreben 20 verschoben werden. Bei einer zirkulären Belastung beispielsweise werden die Schrägstreben 19 bzw. die Ringe 18 zusammengedrückt. Der Raum zwischen den Schrägstreben 19 verkleinert sich hierdurch. Es kommt zwar zu einer Längenänderung in den aneinanderschließenden Schrägstreben 19, diese wird jedoch nicht auf die Axialstreben 20 übertragen.

Weiterhin ist zu erkennen, daß die Schrägstreben 19 über an ihren Außenseiten 24 gerundete Abschnitte 25 ineinander übergehen.

Auch beim Stent 16 sind die Knotenpunkte 26 zwischen Schrägstreben 19 und Axialstreben 20 materialmäßig verstärkt ausgebildet. So wird eine Spannungskonstanz in den Knotenpunkten 26 erreicht.

Die Figur 6 zeigt ein Stützgerüst 27, bei dem Schrägstreben 28 so ausgestellt sind, daß sie im Mantel 29 über dessen Umfang 30 verteilt Vorsprünge 31 ausformen. Die Biegungen 32 zwischen den einzelnen Schrägstreben 28 sind hierzu derart über den Umfang 30 nach außen gebogen, daß aus dem Mantel 29 die Vorsprünge 31 herausgebildet werden. Diese gewährleisten eine sichere Haltung eines Stents in einer Körperröhre oder einem Blutgefäß.

Aus der Figur 7 geht ein Stent 33 hervor mit einem Mantel 34, der drei Radialabschnitte 35, 36, 37 aus jeweils einem unterschiedlichen Polymerwerkstoff aufweist.

Auf die Darstellung eines Stützgerüstes ist aus Übersichtlichkeitsgründen verzichtet worden.

Je nach Werkstoffeigenschaften weist der Stent 33 dann beispielsweise in einem Radialabschnitt 36 ein steiferes Verhalten auf als in den beiden Radialabschnitten 35, 37. So ist es möglich, einen Speiseröhrenstent nur in dem Längenbereich zu versteifen, in dem ein Tumor sitzt, an anderen Stellen aber eine Peristaltik (Schluckakt) zuzulassen.

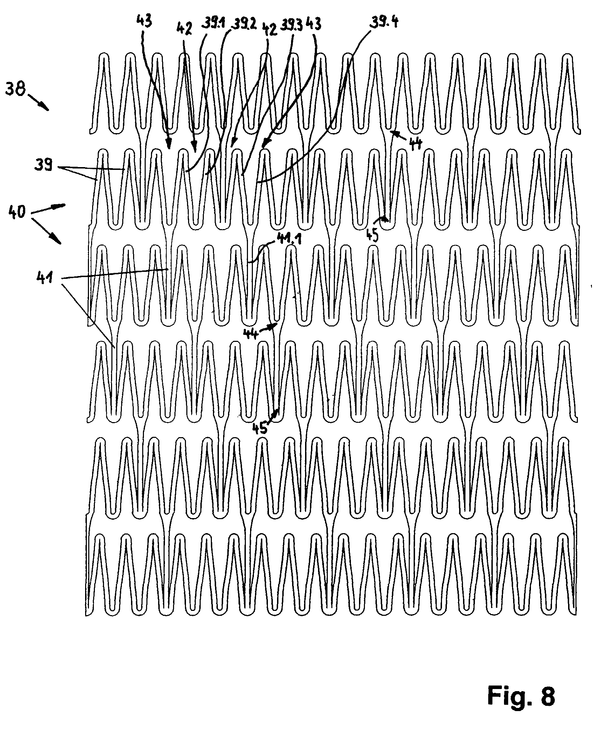

Einen weiteren Aufbau eines Stützgerüstes 38 zeigt die Figur 8. Hierbei bilden wiederum endlos zick-zackförmig aneinanderschließende Schrägstreben 39 Ringe 40, die durch Axialstreben 41 verbunden sind.

Die Ringe 40 sind so aufgebaut, daß sich zwischen zwei von Schrägstreben 39.1, 39.2 gebildeten V-Elementen 42 jeweils ein Y-Element 43 aus zwei Schrägstreben 39.3, 39.4 und eine Axialstrebe 41.1 eingliedert.

Die Axialstreben 41, 41.1 erstrecken sich jeweils von den Spitzen 44 zwischen den Schrägstreben 39.3, 39.4 eines Rings 40 in das Tiefste 45 zwischen den Schrägstreben 39.1, 39.2 des nächsten Rings 40.

Man erkennt ferner, daß die Schrägstreben 39, 39.1-39.4 einen langgestreckten S-förmigen Verlauf besitzen. Dies ist vorteilhaft für die Erzeugung der federnden Rückstellkräfte.

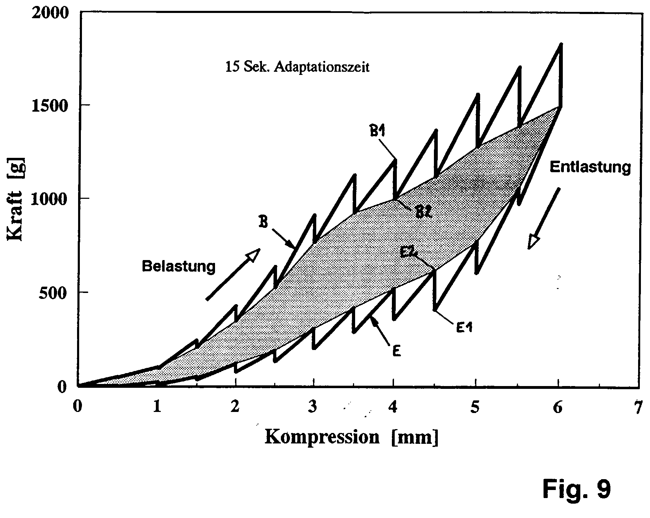

In der Figur 9 ist ein Kraft-Weg-Verlauf eines Stents mit einem Stützgerüst aus Nitinol und einem Mantel aus einem Shape-Memory-Polyurethan veranschaulicht.

Der Stent ist schrittweise stärker zusammengedrückt worden. Dieser Weg ist in Millimetern auf der X-Achse aufgetragen. Die ermittelten Kraftwerte sind als Gewichtskraft gemessen und in Gramm wiedergegeben.

Während der gesamten Kompression bleibt der Stent längenstabil.

Einer äußeren Belastung bringt der Stent anfangs einen höheren Widerstand entgegen. Zeitabhängig sinken die Kraftwerte anschließend ab. Dieser Kraftverlauf ist durch die obere Linie B verdeutlicht.

Andererseits baut der Stent Kraft auf, wenn er entlastet wird. Dies wird durch die untere Kurve E deutlich. Unter kurzfristiger Abnahme der Kompression sinkt zunächst die Rückstellkraft und steigt dann über die Zeit wieder an.

Im hier dargestellten Beispiel beträgt die Zeit zwischen den jeweiligen gemessenen oberen Kraftwerten B1 bzw. E2 und unteren Kraftwerten B2, E1 15 Sekunden.

Der Stent weist somit viskoplastoelastische Eigenschaften auf mit einem dämpfenden Verhalten bei kurzen Belastungen, jedoch einem adaptiven Verhalten bei längerfristigen Belastungsvorgängen.

### Bezugszeichenaufstellung

- 1 -: Stent
- 2 -: Stützgerüst
- 3 -: Mantel
- 4 -: Ring
4.1 - Ring
4.2 - Ring
4.3 - Ring
- 5 -: Schrägstrebe
5.1 - Schrägstrebe
5.2 - Schrägstrebe
5.3 - Schrägstrebe
5.4 - Schrägstrebe
- 6 -: Axialstrebe
6.1 - Axialstrebe
6.2 - Axialstrebe
6.3 - Axialstrebe
- 7 -: Spitze zw. 5.1 u. 5.2
- 8 -: Tiefste zw. 5.3 u. 5.4
- 9 -: Übergang
- 10 -: Schnittbahn
- 11 -: Y-Element
- 12 -: Fuß
- 13 -: V-Element
- 14 -: Knotenpunkt
- 15 -: Knotenpunkt
- 16 -: Stent
- 17 -: Stützgerüst
- 18 -: Ring
- 19 -: Schrägstrebe
19.1 - Schrägstrebe
19.2 - Schrägstrebe
- 20 -: Axialstrebe
- 21 -: Längsträger
- 22 -: Ende v. 16
- 23 -: Ring
- 24 -: Außenseite
- 25 -: Abschnitt
- 26 -: Knotenpunkt
- 27 -: Stützgerüst
- 28 -: Schrägstreben
- 29 -: Mantel
- 30 -: Umfang
- 31 -: Vorsprünge
- 32 -: Biegung
- 33 -: Stent
- 34 -: Mantel
- 35 -: Radialabschnitt v. 1
- 36 -: Radialabschnitt v. 1
- 37 -: Radialabschnitt v. 1
- 38 -: Stützgerüst
- 39 -: Schrägstreben
39.1 - Schrägstrebe
39.2 - Schrägstrebe
39.3 - Schrägstrebe
39.4 - Schrägstrebe
- 40 -: Ringe
- 41 -: Axialstreben
41.1 - Axialstrebe
- 42 -: V-Element
- 43 -: V-Element
- 44 -: Spitze
- 45 -: Tiefste
- A -: Stentachse
- B -: Belastungskurve
- B1 -: oberer Kraftwert bei einer Belastung
- B2 -: unterer Kraftwert bei einer Belastung
- E -: Entlastungskurve
- E1 -: unterer Kraftwert bei einer Entlastung
- E2 -: oberer Kraftwert bei einer Entlastung
- L -: Länge v. 1
- Lₐ -: Längenabschnitt v. 16
- L_{b} -: Längenabschnitt v. 16
- L_{G} -: Länge v. 16

## Patentansprüche

1. Stent zum Anordnen in einer Körperröhre, der als tubuläres netzartiges Stützgerüst (2, 17, 27, 38) aus Metall gestaltet ist, welches aus sich in mehreren zueinander parallelen Ringen (4, 4.1-4.3, 18, 40) endlos zick-zack-förmig aneinanderschließenden Schrägstreben (5, 5.1-5.4, 19, 19.1, 19.2, 28, 39, 39.1-39.4) sowie über sich parallel zur Stentachse (A) erstreckende benachbarte Ringe (4, 4.1-4.3, 18, 40) verbindende Axialstreben (6, 6.1-6.3, 20, 41, 41.1) zusammensetzt, wobei das Stützgerüst (2, 17, 27, 38) durch Schlitzen und anschließendes Aufweiten eines metallischen Tubus gebildet ist und die Axialstreben (6, 6.1-6.3, 20, 41, 41.1) sich jeweils von der Spitze (7, 44) zweier V-förmig aneinanderschließender Schrägstreben (5.1, 5.2, 39.3, 39.4) eines Rings (4.1, 40) in das Tiefste (8, 45) zweier V-förmig aneinanderschließender Schrägstreben (5.3, 5.4, 39.1, 39.2) eines benachbarten Rings (4.2, 4.3, 40) erstrecken.

2. Stent nach Anspruch 1, **dadurch gekennzeichnet**, daß die Axialstreben (20) hintereinander angeordnet sind.

3. Stent nach Anspruch 1, **dadurch gekennzeichnet**, daß die Axialstreben (6.1) eines Rings (4.1) und die Axialstreben (6.2, 6.3) der benachbarten Ringe (4.2, 4.3) in Umfangsrichtung versetzt angeordnet sind.

4. Stent nach einem der Ansprüche 1 bis 3, **dadurch gekennzeichnet**, daß die Schrägstreben (19) dünner als die Axialstreben (20) sind.

5. Stent nach einem der Ansprüche 1 bis 4, **dadurch gekennzeichnet**, daß die Knotenpunkte (14, 15, 26) der Axialstreben (6, 20) mit den Schrägstreben (5, 19) materialmäßig verstärkt ausgebildet sind.

6. Stent nach einem der Ansprüche 1 bis 5, **dadurch gekennzeichnet**, daß am Umfang (30) verteilt Vorsprünge (31) ausgebildet sind.

7. Stent nach einem der Ansprüche 1 bis 6, **dadurch gekennzeichnet**, daß daß das Stützgerüst (2, 17) aus einem Memory-Metall, insbesondere Nitinol, besteht.

8. Stent nach einem der Ansprüche 1 bis 7, **dadurch gekennzeichnet**, daß das Stützgerüst (2, 17) in einen Mantel (3) aus einem thermoplastischen Polymer, insbesondere einem Memory-Elastomer, eingebettet ist.

9. Stent nach Anspruch 8, **dadurch gekennzeichnet**, daß der Mantel (37) Abschnitte (35, 36, 37) aus unterschiedlichen Polymerwerkstoffen aufweist.
